# EUROPÄISCHE PATENTANMELDUNG

(11) **EP 0 689 809 A1**
(43) Veröffentlichungstag der Anmeldung: **03.01.1996**
(21) Anmeldenummer: 95105426.1
(22) Anmeldetag: 11.04.1995
(51) Int. Cl.: A61F 5/01

(54) **Kniegelenkorthese**

(30) Priorität: 30.05.1994 DE 4418806
(71) Anmelder: Otto Bock Orthopädische Industrie Besitz- und Verwaltungs-KG, D-37115 Duderstadt (DE)
(72) Erfinder: Zepf, Armin, D-37115 Duderstadt (DE)
(74) Vertreter: Gramm, Werner, Prof., Dipl.-Ing.

(57) **Zusammenfassung**

Die Erfindung betrifft eine Kniegelenkorthese mit einer über Bandagen oder dergleichen am Oberschenkel des Patienten festzulegenden Oberschenkelschale (2), einer ebenfalls über Bandagen oder dergleichen am Unterschenkel festzulegenden Unterschenkelschale (4) und mit zwei sich seitlich gegenüberliegenden, bei gestrecktem Bein angenähert lotrechten, die beiden Schenkelschalen (2,4) gelenkig miteinander verbindenden Gelenkschienen (5), die jeweils an ihrem oberen Ende über einen oberen Anlenkpunkt (8) mit der Oberschenkelschale (2) und an ihrem unteren Ende über einen unteren Anlenkpunkt (10) mit der Unterschenkelschale (4) so verbunden sind, daß sich der Abstand der beiden Anlenkpunkte (8,10) voneinander von einem maximalen Wert bei gestrecktem Bein auf einen minimalen Wert bei vollständiger Kniebeugung verringert. Zur Vereinfachung des Aufbaus und der Wirkungsweise wird erfindungsgemäß vorgeschlagen, daß die Verschwenkung zumindest einer Gelenkschiene (5) um ihren unteren Anlenkpunkt (10) aus der gestreckten Beinlage in eine Kniebeugung gegen eine der Verschwenkung entgegenstehende Kraft erfolgt, die bis zu einer Kniebeugung von etwa 30° bis 40° ansteigt und bei etwa 90° Beugung (Sitzposition) wieder reduziert ist.

## Beschreibung

Die Erfindung betrifft eine Kniegelenkorthese mit einer über Bandagen oder dergleichen am Oberschenkel des Patienten festzulegenden Oberschenkelschale, einer ebenfalls über Bandagen oder dergleichen am Unterschenkel festzulegenden Unterschenkelschale und mit zwei sich seitlich gegenüberliegenden, bei gestrecktem Bein angenähert lotrechten, die beiden Schenkelschalen gelenkig miteinander verbindenden Gelenkschienen, die jeweils an ihrem oberen Ende über einen oberen Anlenkpunkt mit der Oberschenkelschale und an ihrem unteren Ende über einen unteren Anlenkpunkt mit der Unterschenkelschale so verbunden sind, daß sich der Abstand der beiden Anlenkpunkte voneinander von einem maximalen Wert bei gestrecktem Bein auf einen minimalen Wert bei vollständiger Kniebeugung verringert.

Eine derartige Ausführungsform läßt sich der EP 0 567 673 A1 entnehmen. Bei der hier offenbarten Ausführungsform ist jede der beiden Gelenkschienen teleskopförmig längenveränderlich ausgebildet. Ferner ist eine eine Dorsalierung des Unterschenkels über die Rückverlagerung der Gelenkschienen bewirkende Einrichtung vorgesehen, die durch eine ventrale elastische Zuggurtung gebildet ist, die um die seitlichen Gelenkschienen geführt wird. Jede Gelenkschiene besteht aus zwei teleskopförmig ineinandergeführten Teleskopstangen, die über einen inneren Gummizug miteinander verbunden sein können. Die beiden unteren Anlenkpunkte der Gelenkschienen sind über einen dorsalen Bügel miteinander verbunden.

Ein wesentliches Merkmal dieser vorbekannten Orthese ist darin zu sehen, daß kein mechanisches, die individuelle Kniegelenkmechanik nachahmendes Gelenk mehr vorgesehen ist. Vielmehr wird der Kniegelenkmechanik freier Raum gelassen, um die physiologische Bewegung ungehindert durchführen zu können. Der jeweilige Bewegungsmittelpunkt, mit dem auch seitlich unterschiedlichen Verlauf der Gelenkachse, kann sich entsprechend den individuellen anatomischen Verhältnissen zwischen den Anlenkpunkten der Gelenkschienen einstellen. Durch die Verbindung von Oberschenkel- und Unterschenkelschale wird ein Abhebeln oder Verrutschen und Scheuern aufgrund einer eigenständigen Orthesengelenkmechanik vermieden. Jede Schale weist dorsal im Bereich der Kniekehle einen Ausschnitt auf, um eine starke Beugung des Knies bis zur Anlage des Unterschenkels am Oberschenkel zu ermöglichen.

Der Erfindung liegt die Aufgabe zugrunde, die eingangs beschriebene Kniegelenkorthese in ihrem Aufbau zu vereinfachen und in ihrer Wirkungsweise noch weiter zu verbessern.

Diese Aufgabe wird erfindungsgemäß dadurch gelöst, daß die Verschwenkung zumindest einer Gelenkschiene um ihren unteren Anlenkpunkt aus der gestreckten Beinlage in eine Kniebeugung gegen eine der Verschwenkung entgegenstehende Kraft erfolgt, die bis zu einer Kniebeugung von etwa 30° bis 40° ansteigt und bei etwa 90° Beugung (Sitzposition) wieder reduziert ist.

Erfindungsgemäß wird somit bei Beginn der Kniebeugung, also bei Verschwenkung der Gelenkschienen ein Beugewiderstand aufgebaut; das Abknicken der Gelenkschiene ist somit nur unter einem definierten Kraftaufwand möglich. Diese Kraft, die vorzugsweise justierbar ist, beschreibt den distalisierenden Druck der Unterschenkelschale der Orthese auf den Tibiakopf. Zur Unterstützung der vorderen Kreuzbandfunktion wird der Druckmechanismus auf die ventralen Tibiaanteile bei leichter Kniebeugung aufgebaut, erreicht bei 30° bis 40° seine größte Wirkung und ist bei 90° Kniebeugung, also in Sitzposition, wieder reduziert. Damit wird eine günstige Grundeinstellung des kreuzbandgeschädigten Kniegelenkes in leichter Flexionsstellung gewährleistet. Gerade in dieser Position treten üblicherweise Instabilitäten auf, die zum Wegknicken des Kniegelenkes führen und als "Giving-Way" bezeichnet werden.

In einer bevorzugten Ausführungsform ist die zumindest eine Gelenkschiene mit einer Nockenrolle oder dergleichen bestückt, die von einer federelastischen Kraft gegen einen an der Unterschenkelschale sitzenden Nocken gedrückt wird. Dabei steigt die Nockenkurve von einem die angenähert lotrechte Stellung der Gelenkschiene definierenden Abschnitt auf eine Nockenspitze an, die bei einem Verschwenkwinkel der Gelenkschiene von 30° bis 40° liegt. In einer speziellen konstruktiven Lösung ist es vorteilhaft, wenn die Gelenkschiene aus einem Rohr besteht, das in einer den oberen Anlenkpunkt bildenden Führung längsverschieblich geführt ist und eine in ihr längsverschieblich gelagerte Stößelstange umschließt, die an ihrem unteren Ende mit der Nockenrolle bestückt ist und sich mit ihrem oberen Ende gegen eine vorgespannte Feder abstützt.

Die Beugebewegung des natürlichen Kniegelenkes wird somit durch eine Kombination von Verkürzung und Auslenkung des Abstandes der beiden Anlenkpunkte realisiert, deren jeweilige Verbindung jedoch starr ausgeführt ist. Ein mechanisches Gelenk, eine Gelenkachse und damit ein mechanischer Drehpunkt sind somit nicht vorhanden.

Der obere Anlenkpunkt der Gelenkschiene ist erfindungsgemäß proximal zum Kniedrehbereich angeordnet und liegt vorzugsweise 30 bis 40 mm oberhalb des physiologischen Kniedrehpunktes. Bei Flexion erfolgt dadurch eine Rückverlagerung der Gelenkschienen im Bereich des Kniegelenkes, wobei auch bei starker Flexion eine Verspannung der Orthese durch die Nockenmechanik verhindert wird.

Durch die rotationsbewegliche und längsverschiebliche Anordnung des oberen Endes der Gelenkschiene an der Oberschenkelschale ist gewährleistet, daß Ober- und Unterschenkelschale bei der gesamten Bewegung in Streckung und Beugung des Kniegelenkes und bei Rotation keine Zwangsführung oder Verspannung erfahren. Vielmehr erzeugt die Orthese lediglich eine dorsalierende Druckwirkung an der vorderen Tibiakante. Die bei anderen Orthesen auftretenden unphysiologischen Zwangsführungen werden vermieden; es wird eine gute Sicherung der Tibia gegen anteriore Translation erreicht.

Zur leichten Montage des oberen Endes der Gelenkschiene an dem ihr zugeordneten oberen Anlenkpunkt ist es vorteilhaft, wenn die Führung als im Querschnitt angenähert halbkreisförmiges Klipsteil ausgebildet ist, in das das die Beinschiene bildende Rohr nur eingedrückt zu werden braucht.

Zur Steigerung des Komforts ist es vorteilhaft, wenn die die Nockenrolle gegen den Nocken drückende federelastische Kraft mit einem integrierten Dämpfer versehen ist. Ein derartiger Dämpfer kann mit Öl oder Gas arbeiten und dämpft die Bewegung der Stößelstange.

Es wäre möglich, den vorstehend genannten Nocken einteilig mit der Unterschenkelschale auszubilden. Aus Festigkeitsgründen erscheint es jedoch vorteilhaft, wenn der Nocken das obere Ende einer starr mit der Unterschenkelschale verbundenen Schiene darstellt.

Es ist vorteilhaft, wenn die beiden Schenkelschalen zu ihrer Lagesicherung eine Innenauskleidung aufweisen. Hierfür kann die Orthese in Zweischalentechnik konstruiert sein, wobei die äußere Schale die designgerechte Gestalt der Orthese bildet, während die innere Schale als Polsterung gearbeitet ist. Die Außenschale wird vorzugsweise aus einem thermoplastischen Kunststoff in gespritzter Ausführung hergestellt, so daß Verstärkungsrippen und Funktionsteile integriert werden können. Die Polsterung besteht zweckmäßig aus einem geprägten PE-Schaum. Die Flexibilität der Orthesenschalen ist so gewählt, daß sie sich bewegungsbedingten Muskelvolumenveränderungen anpassen können, ohne Druckstellen zu verursachen. Aufgrund der neuartigen Verbindung der beiden Schalen miteinander können die Orthesenschäfte sehr kurz gehalten werden. Hierdurch ergibt sich ein auch eine maximale Flexion gewährleistendes freies Bewegungsausmaß.

Um ein Verrutschen der oberen Schale aufgrund der konischen Anatomie des Oberschenkels nach unten zu verhindern, ist es vorteilhaft, wenn die obere Schale auf ihrer Innenseite oberhalb der innenseitigen Oberschenkelkondylen eine Kondylenbettung aufweist. Diese kann als separate, individuell positionierbare Pelotte ausgebildet sein, die sich zum Beispiel über einen Klettverschluß an der gewünschten Position festlegen läßt. Es besteht aber auch die Möglichkeit, den entsprechenden Innenbereich der oberen Schale verformbar auszubilden, um ihn so an die individuellen Gegebenheiten anpassen zu können. Hierfür kann eine Wasser- oder Gasfüllung vorgesehen werden, die auch selbstregulierend ausgebildet werden kann.

Die erfindungsgemäße Orthese gibt die Möglichkeit, den individuellen, anatomisch vorgegebenen Bewegungsablauf des Kniegelenkes ungestört zu lassen und lediglich den Kraftaufbau an der Vorderseite des Tibiakopfes gelenkspaltnah zu verwirklichen. Als Versorgungsindikation gelten für die erfindungsgemäße Kniegelenkorthese vor allem eine frische vordere Kreuzbandverletzung, bei Restinstabilitäten eine Verwendung beim Sport und bei nicht operativ versorgten Kreuzbandinstabilitäten als Schutz.

Weitere Merkmale der Erfindung sind Gegenstand der Unteransprüche und werden in Verbindung mit weiteren Vorteilen der Erfindung anhand von Ausführungsbeispielen näher erläutert.

In der Zeichnung sind zwei als Beispiele dienende Ausführungsformen der Erfindung dargestellt. Es zeigen:

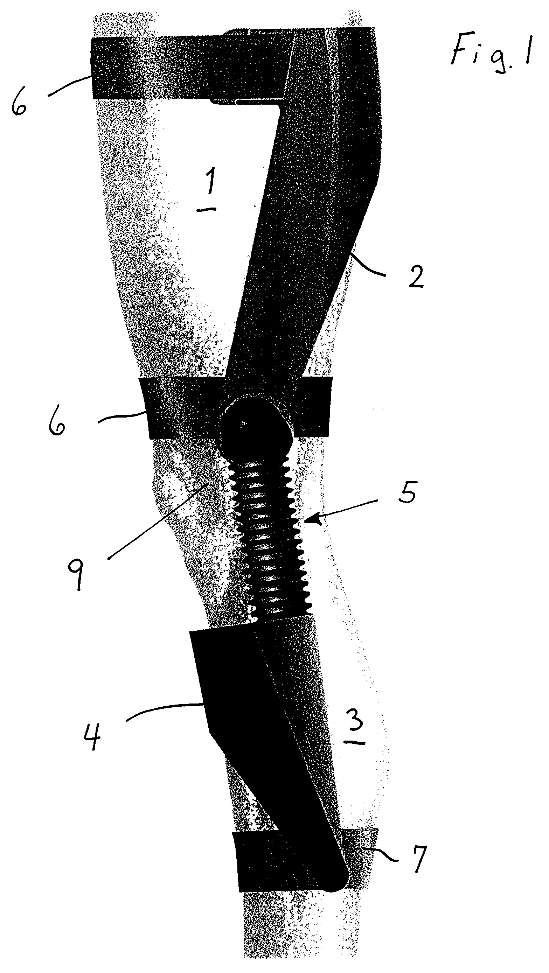

- **Figur 1 -**: in Seitenansicht eine an einem gestreckten Bein festgelegte Kniegelenkorthese;

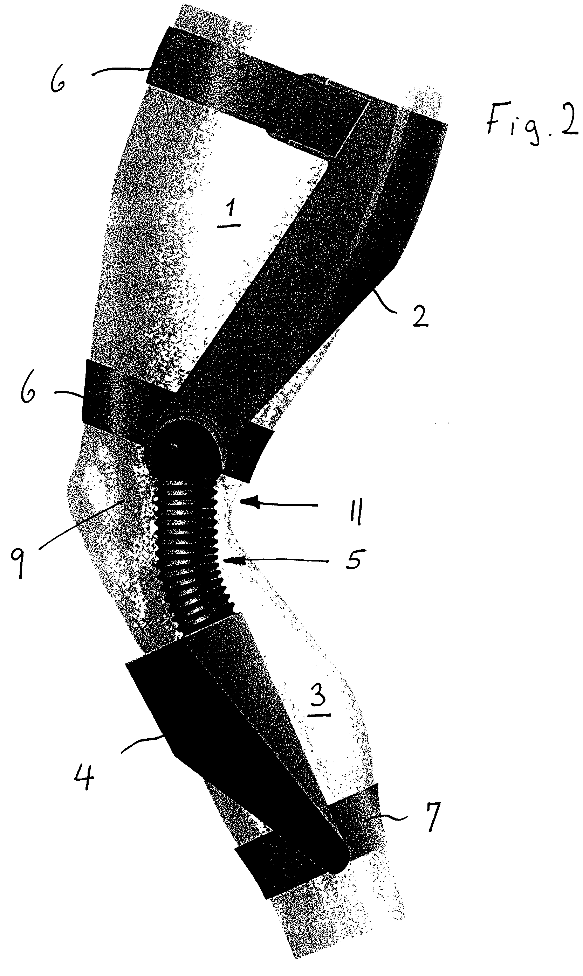

- **Figur 2 -**: die Darstellung gemäß Figur 1 bei gebeugtem Knie;
- **Figur 3 -**: in einer Darstellung gemäß Figur 1 die Orthese in Alleinstellung mit einem Längsschnitt durch eine Gelenkschiene;
- **Figur 4 -**: die Gelenkschiene gemäß Figur 3 in abgewandelter Ausführungsform;
- **Figur 5 -**: die Gelenkschiene gemäß Figur 4 in abgewinkelter Position und
- **Figur 6 -**: ein Diagramm über die wirksamen Andruckkräfte.

Die dargestellte Kniegelenkorthese umfaßt eine am Oberschenkel 1 festzulegende Oberschenkelschale 2, eine am Unterschenkel 3 festzulegende Unterschenkelschale 4 und zwei sich seitlich gegenüberliegende, bei gestrecktem Bein angenähert lotrechte, die beiden Schenkelschalen 2, 4 gelenkig miteinander verbindende Gelenkschienen 5. Die Oberschenkelschale 2 umgreift den Oberschenkel 1 dorsal, ist ventral offen und in diesem Bereich durch Klettverschlüsse 6 verschließbar, während die Unterschenkelschale 4 den Unterschenkel 3 ventral umgreift, dorsal offen ist und hier durch einen Klettverschluß 7 verschließbar ist.

Jede Gelenkschiene 5 ist an ihrem oberen Ende über einen oberen Anlenkpunkt 8 mit der Oberschenkelschale 2 und an ihrem unteren Ende über einen unteren Anlenkpunkt 9 mit der Unterschenkelschale 4 so verbunden, daß sich der Abstand der beiden Anlenkpunkte 8, 9 voneinander von einem maximalen Wert bei gestrecktem Bein auf einen minimalen Wert bei vollständiger Kniebeugung verringert. Dabei ist der obere Anlenkpunkt 8 proximal zum Kniedrehbereich 9 angeordnet, liegt also oberhalb des physiologischen Kniedrehpunktes, während der untere Anlenkpunkt 10 der Gelenkschiene 5 im Bereich des Kniegelenkspaltes 11 liegt. Die bei gestrecktem Bein innerhalb der Sagittalebene des Beines etwa mittig zum Bein liegenden Gelenkschienen 5 bewegen sich dadurch bei zunehmender Kniebeugung hinter den Kniedrehbereich 9. Der lotrechte Abstand des oberen Anlenkpunktes 8 vom physiologischen Kniedrehpunkt beträgt vorzugsweise 30 bis 40 mm. Diese Rückverlagerung der Gelenkschienen 5 bei Flexion, die auf die Unterschenkelschale 4 übertragen wird, ist ein wesentliches Wirkprinzip der Orthese.

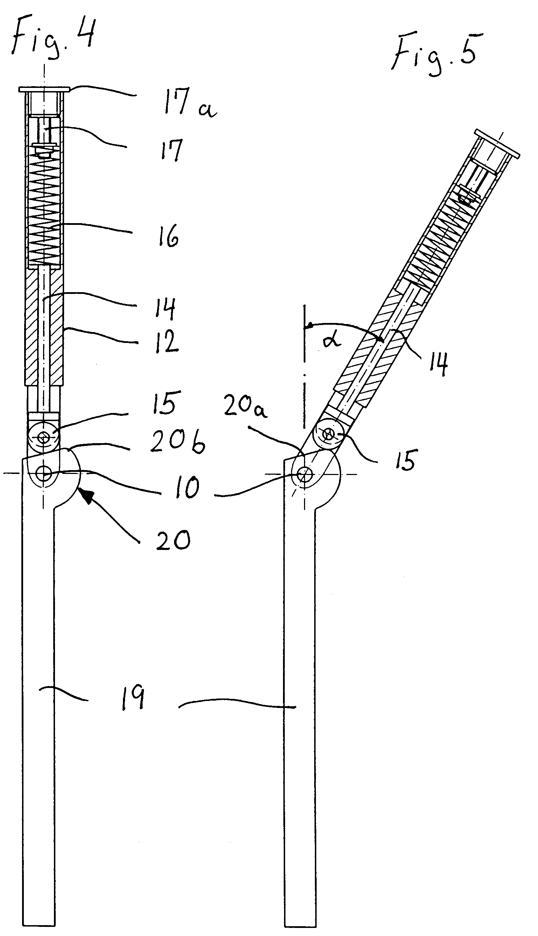

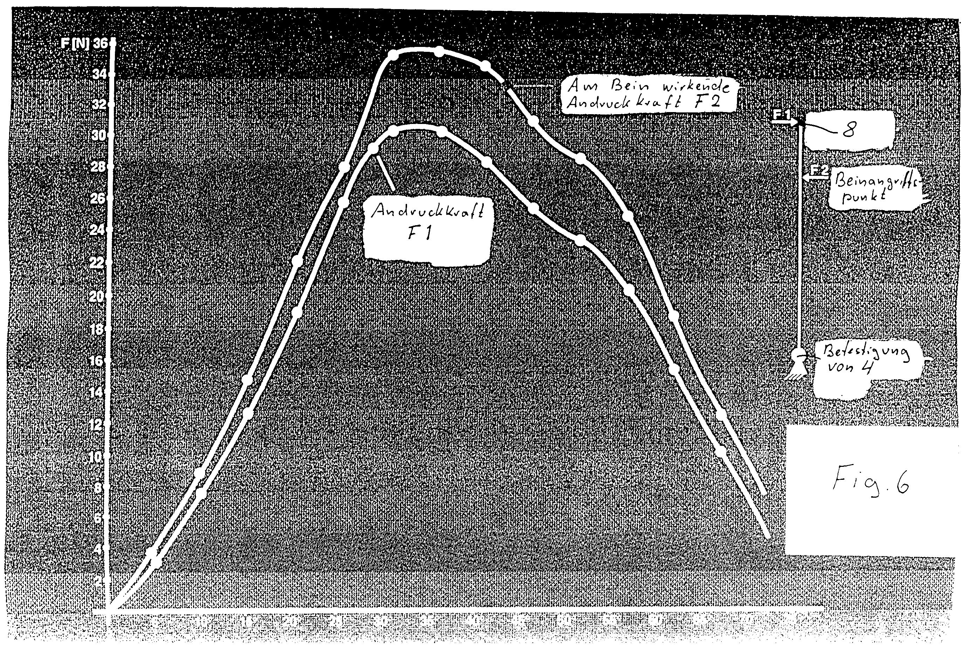

Die Gelenkschiene 5 besteht aus einem Rohr 12, das mit seinem oberen Ende in einer Führung 13 längsverschieblich geführt ist, die als im Querschnitt angenähert halbkreisförmiges Klipsteil ausgebildet ist und zugleich den oberen Anlenkpunkt 12 bildet. Das Rohr 12 umschließt eine in ihr längsverschieblich gelagerte Stößelstange 14, die an ihrem unteren Ende mit einer Nockenrolle 15 bestückt ist und sich mit ihrem oberen Ende gegen eine vorgespannte Feder 16 abstützt. Das obere Ende des Rohres 12 ist durch eine Justierschraube 17 verschlossen, die ein axial verstellbares Widerlager für die Feder 16 bildet, und deren Kopf 17a zugleich einen Anschlag gegenüber der Führung 13 bildet (siehe insbesondere Figuren 4 und 5).

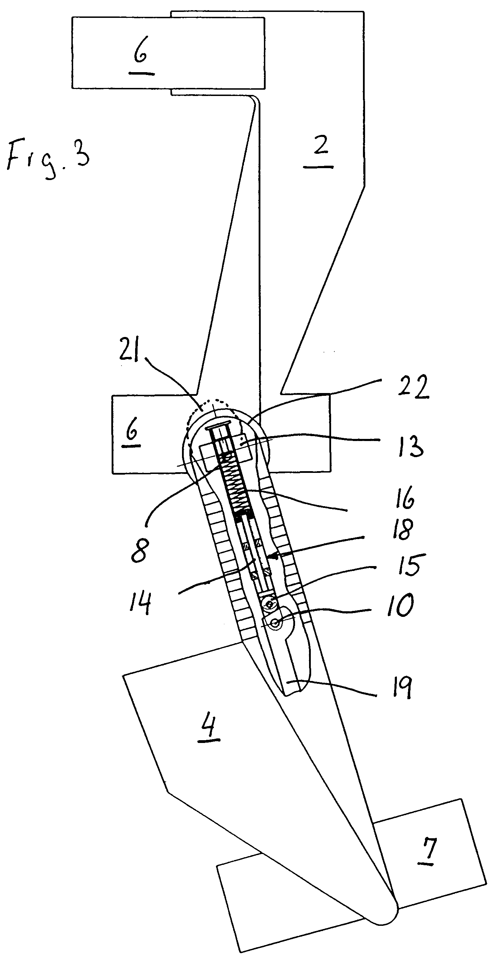

Bei der in Figur 3 dargestellten Gelenkschiene 5 ist die Feder 16 mit einem integrierten Dämpfer 18 versehen, der mit Öl oder Gas arbeitet und die Bewegung der Stößelstange 14 dämpfend steuert.

In den dargestellten Ausführungsbeispielen befindet sich der untere Anlenkpunkt 10 der Gelenkschiene 5 am oberen Ende einer starr mit der Unterschenkelschale 4 verbundenen Schiene 19, deren freies oberes Ende als Nocken 10 ausgebildet ist, gegen den die Nockenrolle 15 mit der von der Feder 16 aufgebrachten Kraft gedrückt wird. Insbesondere die Figuren 4 und 5 lassen erkennen, daß die Nockenkurve von einem die angenähert lotrechte Stellung der Gelenkschiene 5 definierenden Abschnitt 20a auf eine Nockenspitze 20b ansteigt, die bei einem Verschwenkwinkel α der Gelenkschiene 5 von 30° bis 40° liegt.

Das in Figur 6 dargestellte Diagramm zeigt den Verlauf der in Newton angegebenen Federkraft F über den Verschwenkwinkel α der Gelenkschienen 5, also über die Kniebeugung ausgehend von der gestreckten Beinlage mit dem Winkel α = 0. Die untere Kurve zeigt den Verlauf der Andruckkraft F1 am oberen Anlenkpunkt 8 der Führung 13 und die obere Kurve die am Beinangriffspunkt (Kniedrehbereich 9) wirkende Andruckkraft F2. Durch dieses Diagramm wird deutlich, daß sich bei Beginn einer Kniebeugung und damit einer Verschwenkung der Gelenkschienen 5 ein zunehmender Beugewiderstand aufbaut; das Abknicken der Gelenkschienen 5 ist somit nur unter einem definierten Kraftaufwand möglich. Diese Kraft, die sich mit Hilfe der Justierschraube 17 regulieren läßt, ergibt sich aus der Federkraft und Federkennlinie der Feder 16, aus der Form des Nocken 20 sowie aus dem Hebelverhältnis, das durch die Anlenkpunkte 8, 10 definiert wird.

Die Oberschenkelschale 2 kann auf ihrer Innenseite oberhalb der innenseitigen Oberschenkelkondyle eine Kondylenbettung 21 aufweisen, die bei der Darstellung gemäß Figur 3 etwa hinter dem kreisförmigen Bereich 22 liegt und als separate, individuell positionierbare Pelotte ausgebildet oder aber durch einen verformbaren Bereich gebildet sein kann.

Zu ihrer Lagesicherung sind die beiden Schenkelschalen 2, 4 mit einer in der Zeichnung nicht näher dargestellten Innenauskleidung versehen.

## Patentansprüche

1. Kniegelenkorthese mit einer über Bandagen oder dergleichen am Oberschenkel (1) des Patienten festzulegenden Oberschenkelschale (2), einer ebenfalls über Bandagen oder dergleichen am Unterschenkel (3) festzulegenden Unterschenkelschale (4) und mit zwei sich seitlich gegenüberliegenden, bei gestrecktem Bein angenähert lotrechten, die beiden Schenkelschalen (2,4) gelenkig miteinander verbindenden Gelenkschienen (5), die jeweils an ihrem oberen Ende über einen oberen Anlenkpunkt (8) mit der Oberschenkelschale (2) und an ihrem unteren Ende über einen unteren Anlenkpunkt (10) mit der Unterschenkelschale (4) so verbunden sind, daß sich der Abstand der beiden Anlenkpunkte (8,10) voneinander von einem maximalen Wert bei gestrecktem Bein auf einen minimalen Wert bei vollständiger Kniebeugung verringert, **dadurch gekennzeichnet**, daß die Verschwenkung zumindest einer Gelenkschiene (5) um ihren unteren Anlenkpunkt (10) aus der gestreckten Beinlage in eine Kniebeugung gegen eine der Verschwenkung entgegenstehende Kraft erfolgt, die bis zu einer Kniebeugung von etwa 30° bis 40° ansteigt und bei etwa 90° Beugung (Sitzposition) wieder reduziert ist.

2. Kniegelenkorthese nach Anspruch 1, **dadurch gekennzeichnet**, daß die Größe der genannten Kraft justierbar ist.

3. Kniegelenkorthese nach Anspruch 1 oder 2, **dadurch gekennzeichnet**, daß die zumindest eine Gelenkschiene (5) mit einer Nockenrolle (15) oder dergleichen bestückt ist, die von einer federelastischen Kraft gegen einen an der Unterschenkelschale (4) sitzenden Nocken (20) gedrückt wird.

4. Kniegelenkorthese nach Anspruch 3, **dadurch gekennzeichnet**, daß die Nockenkurve von einem die angenähert lotrechte Stellung der Gelenkschiene (5) definierenden Abschnitt (20a) auf eine Nockenspitze ansteigt, die bei einem Verschwenkwinkel (α) der Gelenkschiene (5) von 30° bis 40° liegt.

5. Kniegelenkorthese nach einem der vorhergehenden Ansprüche, **dadurch gekennzeichnet**, daß der obere Anlenkpunkt (8) der Gelenkschiene (5) proximal zum Kniedrehbereich (9) angeordnet ist.

6. Kniegelenkorthese nach einem der vorhergehenden Ansprüche, **dadurch gekennzeichnet**, daß der untere Anlenkpunkt (10) der Gelenkschiene (5) im Bereich des Kniegelenkspaltes (11) liegt.

7. Kniegelenkorthese nach einem der vorhergehenden Ansprüche, **dadurch gekennzeichnet**, daß die Gelenkschienen (5) bei gestrecktem Bein innerhalb der Sagittalebene des Beines mittig zum Bein liegen und sich bei zunehmender Kniebeugung hinter den Kniedrehbereich (9) bewegen.

8. Kniegelenkorthese nach einem der Ansprüche 3 bis 7, **dadurch gekennzeichnet**, daß die Gelenkschiene (5) aus einem Rohr (12) besteht, das in einer den oberen Anlenkpunkt (8) bildenden Führung (13) längsverschieblich geführt ist und eine in ihr längsverschieblich gelagerte Stößelstange (14) umschließt, die an ihrem unteren Ende mit der Nockenrolle (15) bestückt ist und sich mit ihrem oberen Ende gegen eine vorgespannte Feder (16) abstützt.

9. Kniegelenkorthese nach Anspruch 8, **dadurch gekennzeichnet**, daß das obere Ende des Rohres (12) durch eine Justierschraube (17) verschlossen ist, die ein axial verstellbares Widerlager für die Feder (16) bildet.

10. Kniegelenkorthese nach Anspruch 9, **dadurch gekennzeichnet**, daß der Kopf (17a) der Justierschraube (17) zugleich einen Anschlag gegenüber der Führung (13) bildet.

11. Kniegelenkorthese nach einem der Ansprüche 3 bis 10, **dadurch gekennzeichnet**, daß die die Nockenrolle (15) gegen den Nocken (20) drückende federelastische Kraft mit einem integrierten Dämpfer (18) versehen ist.

12. Kniegelenkorthese nach Anspruch 8, 9 10 oder 11, **dadurch gekennzeichnet**, daß die Führung (13) als im Querschnitt angenähert halbkreisförmiges Klipsteil ausgebildet ist.

13. Kniegelenkorthese nach einem der Ansprüche 3 bis 12, **dadurch gekennzeichnet**, daß der Nocken (20) das obere Ende einer starr mit der Unterschenkelschale (4) verbundenen Schiene (19) darstellt.

14. Kniegelenkorthese nach einem der vorhergehenden Ansprüche, **dadurch gekennzeichnet**, daß die Oberschenkelschale (2) den Oberschenkel (1) dorsal umgreift und ventral durch Klettverschlüsse (6) verschließbar ist, während die Unterschenkelschale (4) den Unterschenkel (3) ventral umgreift und dorsal durch einen Klettverschluß (7) verschließbar ist.

15. Kniegelenkorthese nach einem der vorhergehenden Ansprüche, **dadurch gekennzeichnet**, daß die Oberschenkelschale (2) auf ihrer Innenseite oberhalb der innenseitigen Oberschenkelkondylen eine Kondylenbettung (21) aufweist.

16. Kniegelenkorthese nach Anspruch 15, **dadurch gekennzeichnet**, daß die Kondylenbettung (21) als separate, individuell positionierbare Pelotte ausgebildet ist.

17. Kniegelenkorthese nach Anspruch 15, **dadurch gekennzeichnet**, daß die Kondylenbettung (21) durch einen verformbaren Bereich gebildet ist.

18. Kniegelenkorthese nach einem der vorhergehenden Ansprüche, **dadurch gekennzeichnet**, daß die beiden Schenkelschalen (2,4) zu ihrer Lagesicherung eine Innenauskleidung aufweisen.
